# EUROPEAN PATENT APPLICATION

(11) **EP 4 566 592 A1**
(43) Date of publication of application: **11.06.2025**
(21) Application number: 23849227.6
(22) Date of filing: 21.07.2023
(51) Int. Cl.: A61K 9/20, A61P 37/00, A61P 37/08

(54) **SUBLINGUAL TABLET AND APPLICATION THEREOF IN DESENSITIZATION THERAPY DRUG**

(30) Priority: 02.08.2022 CN 202210920347
(71) Applicant: Zonhon Biopharma Institute, Inc., Changzhou, Jiangsu 213125 (CN)
(72) Inventor: MA, Bruce Yong, Changzhou, Jiangsu 213125 (CN); WANG, Rong, Changzhou, Jiangsu 213125 (CN); WANG, Jun, Changzhou, Jiangsu 213125 (CN); XU, Yinmei, Changzhou, Jiangsu 213125 (CN); WANG, He, Changzhou, Jiangsu 213125 (CN)
(74) Representative: Laufhütte, Dieter
(86) International application number: PCT/CN2023/108667
(87) International publication number: WO 2024/027519

(57) **Abstract**

The present disclosure relates to sublingual tablets and application thereof in desensitization therapy drug. The sublingual tablet of the present disclosure is a low-dose allergen sublingual tablet. The sublingual tablet comprises freeze-dried powder of allergen protein, lactose, mannitol, low substituted hydroxypropyl cellulose, low moisture microcrystalline cellulose, and magnesium stearate. The present disclosure adopts the freeze-drying process to obtain the freeze-dried powder of allergen protein, and adopts the powder direct compression technology to obtain the sublingual tablet of allergen protein. The low-dose sublingual tablet of the present disclosure has excellent properties, including appearance, hardness, tablet weight variation, disintegration time, taste, and uniformity of content, in particular has outstanding advantages in disintegration time, uniformity of content, stability, and preparation process, and has an industrial application prospect.

## Description

### TECHNICAL FIELD

The present disclosure relates to the field of pharmaceutical preparations, and in particular to a sublingual tablet and an application thereof in a desensitization therapy drug.

### BACKGROUND

Allergen specific immunotherapy is the only "causal treatment" method that achieves the goal of treating allergic diseases through immune regulation. By using gradually increasing doses of allergens, it can improve the tolerance of patients to the allergen, reduce the symptoms caused by exposure to the allergen, and finally achieve the purpose of tolerance and even immune tolerance.

Allergen specific immunotherapy mainly includes subcutaneous immunotherapy (SCIT) and sublingual immunotherapy (SLIT). Allergen specific immunotherapy requires a long course of treatment (2-3 years) with multiple subcutaneous injections. Therefore, the compliance of patients is poor, and it is easy to cause a variety of adverse reactions, such as severe systemic anaphylactic shock, poor patient adaptability, and easy to cause a variety of adverse reactions, such as severe systemic anaphylactic shock. Compared with subcutaneous administration, sublingual administration has fewer side effects, higher safety and better compliance.

At present, sublingual administration of allergen mainly includes sublingual drops and sublingual tablets. Sublingual tablets are placed under the tongue and can be rapidly dissolved or disintegrated in saliva, and drugs are absorbed through the sublingual oral mucosa to exert systemic effects. Compared with sublingual drops, sublingual tablets are convenient to take and carry, and the dosage is easily controlled, which can ensure the standardization of dosage. The compliance of patients is higher, and the stability of sublingual tablets is better.

Compared with common sublingual tablets, the formulation development of allergen sublingual tablets is more difficult. Firstly, the active ingredient of allergen sublingual tablets is protein, which is unstable with humidity and heat. Currently, the preparation of common sublingual tablets includes granulation and compression (including wet granulation and dry granulation), freeze-drying in blister packaging and direct compression. Among them, the wet granulation and compression method needs granulation, drying, and sizing, which is not suitable for heat-sensitive, moise-sensitive, and highly soluble drugs. The dry granulation and compression method requires the use of compressive force to generate binding force between particles, but high pressure can easily lead to issues such as crystal form transformation and reduced activity. The marketed allergen sublingual tablets, such as QDACTRA^{®} produced by ALK, use the freeze-drying in blister packaging method, which has high requirements for production equipment, including specialized production lines with liquid nitrogen freezing tubes, resulting in high equipment costs. Moreover, the related technology in China is not yet mature. Overall, the direct powder compression method avoids the wet granulation step and the drying process, making it more suitable for drugs that are unstable under heat and humidity. It also simplifies the overall process, saving time and energy. However, the direct powder compression process has some shortcomings, such as poor powder flowability, large variation in tablet weight, and the tendency to cause tablet cracking and poor content uniformity. In particular, when the active ingredient content in the tablet is low, the problem of poor content uniformity in the direct powder compression process becomes more prominent.

Moreover, compared with ordinary sublingual tablets, the active ingredient content of allergen sublingual tablets is extremely low in the tablets, accounting for less than 2% of the tablet weight, or even 1% or less. This extremely low active ingredient content poses challenges in terms of content uniformity and stability. Chinese Patent CN108524454A discloses the use of twin-screw equipment for the preparation of low-dose drugs; while Chinese Patent CN1531423A adopts a high-shear granulation method for the preparation of low-dose drugs. Similarly, low-dose drug impose additional requirements on production equipment.

### SUMMARY

Addressing the shortcomings of existing technologies, the present disclosure provides a low-dose sublingual tablet suitable for multiple active ingredients and its application in desensitization therapy drugs, while simultaneously resolving issues related to poor content uniformity and stability of low-dose sublingual tablets, as well as issues in production equipment and processes.

The present disclosure provides a sublingual tablet including a protein raw material, lactose, mannitol, low-substituted hydroxypropyl cellulose, low-moisture microcrystalline cellulose, and magnesium stearate, with mass percentages of the protein raw material, the lactose, the mannitol, the low substituted hydroxypropyl cellulose, the low moisture microcrystalline cellulose, and the magnesium stearate are 0%-15.6%, 0%-83.4%, 0-83.4%, 4%-6%, 10%-15%, and 0.4%-0.6%, respectively.

Preferably, the protein raw material is allergen protein raw material.

More preferably, the mass percentages of the protein raw material, lactose, mannitol, low-substituted hydroxypropyl cellulose, low-moisture microcrystalline cellulose, and magnesium stearate are 0%-15.6%, 50%-83.4%, 0%-33.4%, 4%-6%, 10%-15%, and 0.4%-0.6%, respectively.

Even more specifically, the mass percentages of the protein raw material, lactose, mannitol, low-substituted hydroxypropyl cellulose, low-moisture microcrystalline cellulose, and magnesium stearate are 0.276%-15.6%, 59.3%-76.48%, 5%-13.4%, 4%-6%, 10%-12.5%, and 0.4%-0.6%, respectively.

There is a variety of allergens, primarily divided into inhaled allergens and food allergens. Common inhaled allergens include dust mites, cockroaches, cat and dog dander, mold and pollen, while common food allergens include milk, peanuts, and eggs. The allergen proteins mentioned can be naturally extracted or recombinantly expressed. The major allergenic proteins of Dermatophagoides pteronyssinus (Der p) are Der p 1 and Der p 2, while those of Dermatophagoides farinae (Der f) are Der f 1 and Der f 2. The primary allergenic proteins of Artemisia annua pollen are Art a 1 and Art a 3, and those of Artemisia vulgaris pollen are Art v 1 and Art v 3. Due to the extremely low content of active ingredients in the sublingual tablets of the present disclosure, although the physicochemical properties of different active ingredients vary, the data from examples demonstrate that different active ingredients do not affect the final properties of the sublingual tablets such as hardness, weight variation, disintegration time, content uniformity, and stability. Therefore, the sublingual tablet formulation of the present disclosure is suitable for multiple low-dose active ingredients and is not limited to the specific active ingredients listed in the examples.

Preferably, the allergen protein raw materials for dust mites comprises one or a mixture of two or more selected from the group consisting of a Der p 1 raw material, a Der p 2 raw material, a Der f 1 raw material, and a Der f 2 raw material. The allergen protein raw materials for Artemisia annua/vulgaris pollen comprises one or a mixture of two or more selected from the group consisting of an Art a 1 raw material, an Art a 3 raw material, an Art v 1 raw material, and an Art v 3 raw material.. More preferably, the allergen protein raw materials are freeze-dried powders.

The second aspect of the present disclosure relates to a freeze-drying process. The buffer components and protein concentration of the freeze-drying solution suitable for a specific protein can be determined through limited experiments based on conventional methods. The buffer components selected in the specific examples are only illustrative and do not limit the buffer components used for freeze-drying allergen proteins. For example, when the active ingredients are Der p 2 and Der f 2, the buffer components can be PB and mannitol. When the active ingredients are Der p 1, Der f 1, Art a 1, Art a 3, Art v 1, and Art v 3, the buffer components can be citrate-sodium citrate and mannitol. The protein concentration ranges from 0.03 to 1.0 mg/mL. Furthermore, the allergen protein freeze-drying solution can be supplemented with gelatin or not. When the stability of the allergen protein is poor, adding gelatin is more conducive to maintaining the stability of the allergen protein sublingual tablet.

The general process of freeze-drying involves replacing the solution containing allergen proteins with a suitable buffer system with suitable pH condition, adjusting to an appropriate concentration, and undergoing three steps: pre-freezing, frist drying, and secondary drying, to obtain the freeze-dried product. A suitable freeze-drying process for a specific protein can be screened using conventional methods. Case 12-15 enumerate the freeze-drying processes for different allergen proteins, which are only illustrative and do not limit the freeze-drying processes for allergen proteins. For example, different freeze-drying processes are used for dust mite type II proteins in case 12, 16, and 20, yet the final tablet properties are equivalent. The pre-freezing step is divided into two stages: the first stage is set at -12°C to -15°C, maintained for 60-90 minutes after reaching this temperature; the second stage is set at -45°C to -50°C, maintained for 120-800 minutes after reaching this temperature. The first drying temperature is set at -10°C to -20°C, maintained for 840-960 minutes after reaching this temperature, with a vacuum level of 0.18-0.2 mbar. The secondary drying temperature is set at 20°C to 25°C, maintained for 480-1500 minutes after reaching this temperature, with a vacuum level of 0.18-0.2 mbar.

The third aspect of the present disclosure relates to a direct powder compression process for allergen sublingual tablets, which eliminates the need for complex processes and equipment. The direct powder compression method avoids the granulation process, directly compressing a mixture of active drugs and excipients into tablets. The process primarily contains crushing, sieving, blending the main drug and excipients, re-blending with a lubricant, and compression. Through extensive experimentation, the inventor has developed a low-dose sublingual tablet formulation that not only exhibits ideal hardness, weight variation, disintegration time, content uniformity, and stability but is also highly suitable for direct powder compression, overcoming the issues associated with traditional direct powder compression processes.

The allergen sublingual tablets of the present disclosure has excellent properties, including appearance (smooth surface, uniformly white tablets), hardness (moderately hard at 15-60N, with no significant impact on packaging and transportation), weight variation, disintegration time, taste, and content uniformity, in particular has advantages in disintegration time, content uniformity, stability, and preparation process:
1. The sublingual tablets of the present disclosure rapidly disintegrate and dissolve under the tongue with no obvious foreign body sensation, ensuring that active ingredients are absorbed through the sublingual mucosa to exert systemic effects. Prolonged disintegration can lead to swallowing, with some active ingredients failing to be fully absorbed through the sublingual mucosa, resulting in inaccurate dosing.
2. The sublingual tablets exhibit excellent content uniformity of allergens, avoiding the problem of inhomogenous in tablets with low content of active ingredients. It is suitable for allergen sublingual tablets with low active ingredient content.
3. These sublingual tablets are stable not only at room temperature but also under harsher conditions, suitable for various biologically active drugs.
4. With excellent properties, the low-dose sublingual tablets of the present disclosure are prepared through a simple direct powder compression process, requiring minimal steps and no additional requirements for production equipment.

### DETAILED DESCRIPTION OF THE EMBODIMENTS

The following are cases and control groups to elaborate on the present disclosure, but the present disclosure is not limited to them.

Case 1-11 are cases and control groups for blank sublingual tablets without active ingredients. The obtained tablets are tested for tablet hardness, weight variation, disintegration time, and sensory evaluation.

Case 12-26 are sublingual tablets containing low-dose active ingredients. In addition to tests such as tablet hardness, weight variation, disintegration time, content uniformity, and sensory evaluation, the tablets are also tested for long-term stability studies, high temperature test, light test and high-humidity test. Stability is assessed by detecting the total protein content and purity of the sublingual tablets.

Specific testing methods are as follows:

### 1. Hardness Test

Use a hardness tester (e.g., CJY-2C Hardness Tester, Tianda Tianfa) to test the hardness of the tablets. Randomly select 10 tablets for testing, record their average hardness, and calculate the RSD (Relative Standard Deviation) value.

### 2. Weight Variation Test

Follow the Weight Variation Test method in the 2020 Chinese Pharmacopoeia General Chapter 0101: Take 20 tablets, accurately weigh the total weight, calculate the average tablet weight, and then accurately weigh each tablet individually. No more than 2 tablets should exceed the weight variation limit, and none should exceed the limit by more than one time. (For tablets with a nominal or average weight of less than 0.3g, the weight variation limit is ±7.5%.)

### 3. Disintegration Time Test

Follow the Disintegration Test method in the 2020 Chinese Pharmacopoeia General Chapter 0921: Use a disintegrator (e.g., ZB-1E, Tianda Tianfa) and immerse a 1000ml beaker filled with water at 37°C±1°C. Take 6 tablets and test them to ensure that each tablet completely disintegrates and dissolves within 5 minutes.

### 4. Moisture Content Testing of Freeze-Dried Powder

Refer to the Karl Fischer Titration Method in the 2020 Chinese Pharmacopoeia General Chapter 0832 Moisture Determination Method.

### 5. Sensory Evaluation

Select 6 healthy adults to sublingually administer the tablets and evaluate the taste, including dryness or discomfort, gritty sensation, sweetness level, and oral disintegration time. After the test, the tablets should be spat out and the mouth rinsed with water.

### 6. Excipient Compatibility Test

Weigh appropriate amounts of active drug freeze-dried powder into vials and add different proportions of excipients, including microcrystalline cellulose, low-moisture microcrystalline cellulose, fillers, low-substituted hydroxypropyl cellulose (5 times the amount of freeze-dried powder), and lubricants (equal to the amount of freeze-dried powder). Conduct accelerated test, high-humidity test, and light test to confirm the compatibility of the active drug freeze-dried powder with each excipient through purity test.

### 7. Accelerated Test

Place the test samples in vials, seal them, and place the vials in a stability chamber (e.g., KBF-P-720, Binder) under accelerated conditions (40±2°C, 75%±5% RH). After a period of time, remove the samples for testing.

### 8. High-Temperature Test

Place the test samples in vials, seal them, and place the vials in a stability chamber (e.g., KBF-P-720, Binder) under high-temperature conditions (60±2°C). After a period of time, remove the samples for testing.

### 9. High-Humidity Test

Place the test samples in open vials and place the vials in a stability chamber (e.g., KBF-720, Binder) under high-humidity conditions (25±2°C, 75%±5% RH). After a period of time, remove the samples for testing.

### 10. Light Test

Place the test samples in vials, seal them, and place the vials horizontally in a stability chamber (e.g., KBF-P-720, Binder) under light conditions (25±2°C, 60%±5% RH, 4500±500lx). After a period of time, remove the samples for testing.

### 11. Purity Test

Test the purity of Der p 1 and Der f 1 following the Size Exclusion Chromatography method in the 2020 Chinese Pharmacopoeia General Chapter 0514. Test the purity of Der p 2 and Der f 2 following the Reversed-Phase Chromatography method in the 2020 Chinese Pharmacopoeia General Chapter 0512.

### 12. Protein Content and Uniformity Test of Freeze-Dried Powder

Test the protein content and uniformity (RSD) of Der p 1 and Der f 1 following the Size Exclusion Chromatography method in the 2020 Chinese Pharmacopoeia General Chapter 0514. Test the protein content and uniformity (RSD) of Der p 2 and Der f 2 follow the Reversed-Phase Chromatography method in the 2020 Chinese Pharmacopoeia General Chapter 0512.

### 13. Major Allergen Content and Potency Test

Use a double-antibody sandwich ELISA method to detect protein content, where the total content of Type I major allergens in dust mites refers to the sum of Der p 1 and Der f 1, and the total content of Type II major allergens in dust mites refers to the sum of Der p 2 and Der f 2. The total content of Type I major allergens in Artemisia pollen refers to the sum of Art a 1 and Art v 1, and the total content of Type III major allergens in Artemisia pollen refers to the sum of Art a 3 and Art v 3.

Use an inhibition ELISA method to detect potency. Incubate a series of dilutions of the recombinant protein sample with serum pool serum. Add the incubated mixture to ELISA plates pre-coated with recombinant protein, establish a linear relationship between "inhibition rate - dilution factor" and define the biological activity required to reduce serum pool IgE by 50% as 100BU/ml. Calculate the biological activity of the recombinant protein, and to better reflect the trend of activity changes, express the detected value as a percentage of the theoretical labeled amount: (Actual Detected Activity / Theoretical Labeled Value) * 100%.

### 14. Content Uniformity Test

Detect protein content in tablets using the immunodot method and calculate content uniformity following the 2020 Chinese Pharmacopoeia General Chapter 0941 Content Uniformity Test method. According to Chapter 0941, as a sublingual tablet, the L value for this product should be 15. Test the content of 10 tablets, calculate the standard deviation S, calculate the absolute value A of the difference between the labeled amount and the mean value. If A+2.2S is less than 15, the content uniformity can be judged as qualified.

### Example 1: Formulation Study I (Choice of disintegrants)

**Table 1 Ingredient Ratio for case 1**

| **Ingredients** | **The formulation amount (Ratio)** |
|---|---|
| Lactose | 35g (70.0%) |
| Mannitol | 6.7g (13.4%) |
| L-HPC (Low-substituted hydroxypropyl cellulose) | 2g (4.0%) |
| LMC (Low Moisture Microcrystalline Cellulose) | 6g (12.0%) |
| Magnesium stearate | 0.3g (0.6%) |
| The total amount was 50g, and each tablet weighed 50mg | |

The lactose used was FlowLac^{®} sourced from MEGGLE in Germany; the mannitol was 100SD sourced from ROQUETTE in France; the low-substituted hydroxypropyl cellulose (L-HPC ) used was LH-21, with a hydroxypropyl content ranging from 10.0% to 12.9%, purchased from HuZhou ZhanWang Pharmaceutical Co., Ltd. L-HPC with a hydroxypropyl content ranging from 5.0% to 16.0% (calculated on a dry basis) can be used without affecting the properties of the tablets. The low-moisture microcrystalline cellulose VIVAPUR PH112 had a dry weight loss value of ≤1.5%, and was purchased from Shanghai FengHong Pharmaceutical Excipients Technology Co., Ltd.

Case 1: According to the amounts listed in Table 1, lactose was added layer by layer. Add and mix 5% lactose, mannitol, 5% lactose, L-HPC , 5% lactose, low-moisture microcrystalline cellulose, and the remaining 85% lactose sequentially. Finally, magnesium stearate was added and mixed. A small single-punch tablet press was used, the tablet hardness was 20-60N and tablet weight was 50mg.

Control Groups 1-3: In Control Group 1-3, the L-HPC in case 1 was replaced with hydroxypropyl cellulose (SSL-L), croscarmellose sodium, and sodium carboxymethyl starch, respectively, while the types and amounts of other excipients remained unchanged. The preparation methods of Control Groups 1-3 were the same as that of case 1.

The experimental results showed that when L-HPC was used, the sublingual tablets exhibited low RSD values for hardness, small differences in tablet weight, short oral disintegration time, no dryness or gritty sensation in the mouth, and moderate sweetness. When other disintegrants were used, the disintegration time was too long, and even after adjusting the types and amounts of other excipients in the formulation, it was impossible to obtain tablets with excellent comprehensive properties. The specific results were as follows:

**Table 2 Experimental results of case 1 and control group 1-3**

| | **Case 1** | **Control 1** | **Control 2** | **Control 3** |
|---|---|---|---|---|
| Disintegrant | L-HPC | SSL-L | Croscarmellose Sodium | Carboxymethyl starch sodium |
| Hardness | 30N | 39N | 52N | 58N |
| RSD value of hardness | 4.3% | 28.6% | 15.1% | 30.7% |
| Tablet weight variation | ±1.3% | ±5.4% | ±2.4% | ±2.9% |
| Disintegration time | 35S | 280S | 124S | 105S |
| Oral disintegration time | 42S | 300S | 142S | 120S |
| Oral dryness | None | None | None | Sense of dryness |
| Oral gritty sensation | None | None | Gritty sensation | None |
| Sweet taste | Moderate | Moderate | Moderate | Moderate |

### Example 2: Formulation Study II (Selection and Dosage of Fillers)

**Table 3 Ingredient Ratios for case 2-5**

| **Ingredients** | **The formulation amount (Ratio) of** | | | |
|---|---|---|---|---|
| | **Case 2** | **Case 3** | **Case 4** | **Case 4** |
| Lactose | 41.7g (83.4%) | 25g (50.0%) | 13.7g (27.4%) | / |
| Mannitol | / | 16.7g (33.4%) | 28g (56.0%) | 41.7g (83.4%) |
| L-HPC | 2g (4.0%) | 2g (4.0%) | 2g (4.0%) | 2g (4.0%) |
| LMC | 6g (12.0%) | 6g (12.0%) | 6g (12.0%) | 6g (12.0%) |
| Magnesium Stearate | 0.3g (0.6%) | 0.3g (0.6%) | 0.3g (0.6%) | 0.3g (0.6%) |
| Total | 50g | 50g | 50g | 50g |

Case 2 to 5 were prepared according to the ratios listed in Table 3. In case 2, all mannitol in case 1 was replaced with lactose. In case 3 to 5, part or all of the lactose in case 1 was replaced with mannitol, with other ingredients and preparation methods remaining the same as case 1.

The results were shown in Table 4. The lactose ratios in case 1-5 were approximately 70.0%, 83.4%, 50.0%, 27.4%, and 0%, respectively. The RSD values for tablet hardness, tablet weight variations, oral disintegration time, and sensory evaluation of sublingual tablets were all optimal. Among them, case 1 was the best with the shortest disintegration time, followed by case 3, and case 2 was relatively good. Case 4 and 5 had relatively long disintegration times and slightly inferior taste. Therefore, the combination of lactose and mannitol was optimal, and as the proportion of lactose increased, the RSD values for tablet hardness and tablet weight variations were relatively small, the oral disintegration time was short, and there was no dryness, gritty sensation, or excessive sweetness in the mouth.

**Table 4 Experimental Results for case 2-5**

| | **Case 2** | **Case 3** | **Case 4** | **Case 5** |
|---|---|---|---|---|
| Hardness | 38N | 35N | 40N | 44N |
| RSD value of hardness | 8.4% | 4.8% | 12.4% | 10.2% |
| Tablet weight variation | ±2.6% | ±1.9% | ±2.9% | ±2.2% |
| Disintegration time | 62S | 54S | 84S | 88S |
| Oral disintegration time | 70S | 63S | 89S | 96S |
| Oral dryness | None | None | None | None |
| Oral gritty sensation | None | None | Slightly gritty sensation | Slightly gritty sensation |
| Sweet taste | Slightly sweetness | Moderate | Moderate | Moderate |

### Example 3: Formulation Study III (Selection of Low-Moisture Microcrystalline Cellulose/Microcrystalline Cellulose and Lubricants)

**Table 5 Ingredient Ratios for case 6-7**

| **Ingredients** | **The formulation amount (Ratio)** | | |
|---|---|---|---|
| | **Case 6** | **Case 7** | **control 4** |
| Lactose | 35g (70.0%) | 35g (70.0%) | 47.2g (94.4%) |
| Mannitol | 6.7g (13.4%) | 6.7g (13.4%) | / |
| L-HPC | 2g (4.0%) | 2g (4.0%) | 2.5g (5.0%) |
| LMC | / | 6g (12.0%) | / |
| Microcrystalline cellulose | 6g (12.0%) | / | / |
| Magnesium stearate | 0.3g (0.6%) | / | 0.3g (0.6%) |
| Glyceryl behenate | / | 0.3g (0.6%) | / |
| Total | 50g | 50g | 50g |

Case 6 was prepared by replacing the low-moisture microcrystalline cellulose in case 1 with microcrystalline cellulose, with the preparation method the same as case 1.

Case 7 was prepared by replacing magnesium stearate in case 1 with glyceryl behenyl, with the preparation method the same as case 1.

Control Group 4 was prepared according to the ratio in Table 5, without low-moisture microcrystalline cellulose, microcrystalline cellulose, or mannitol,, with the preparation method the same as case 1.

The results were shown in Table 6, indicating that the use of microcrystalline cellulose and glyceryl behenyl resulted in optimal RSD values for tablet hardness, tablet weight variations, oral disintegration time, and sensory evaluation (however, in the excipient compatibility study of case 11-14, microcrystalline cellulose and glyceryl behenyl exhibited poor compatibility with allergen protein). Control Group 4 had severe powder leakage during the tableting process, making the direct powder compression process unsuitable.

**Table 6 Experimental results of case 6-7 and control group 4**

| | **Case 6** | **Case 7** | **Control 4** |
|---|---|---|---|
| Hardness | 46N | 48N | 53N |
| RSD value of hardness | 5.2% | 4.4% | 6.8% |
| Tablet weight variation | ±1.8% | ±1.9% | ±2.5% |
| Disintegration time | 68S | 70S | 83S |
| Oral disintegration time | 80S | 88S | 95S |
| Oral dryness | None | None | None |
| Oral gritty sensation | None | None | None |
| Sweet taste | Moderate | Moderate | Moderate |

### Example 4: Prescription Study IV (excipients ratio study)

**Table 7 Ingredient Ratios for case 8-11**

| **Ingredients** | **Case 8** | **Case 9** | **Case 10** | **Case 11** | **Control 5** | **Control 6** |
|---|---|---|---|---|---|---|
| Lactose | 34g (68.0%) | 33.5g (67.0%) | 38.55g (77.1%) | 35g (70.0%) | 40.425g (80.85%) | 35.25g (70.5%) |
| Mannitol | 6.7g(13.4%) | 6.7g(13.4%) | 4.15g(8.3%) | 6.8g(13.6%) | 4.15g(8.3%) | 6.8g (13.6%) |
| L-HPC | 3g (6.0%) | 2g (4.0%) | 2g (4.0%) | 2g (4.0%) | 2g (4.0%) | 1.75g (3.5%) |
| LMC | 6g (12.0%) | 7.5g(15.0%) | 5g (10.0%) | 6g (12.0%) | 3.125g (6.25%) | 6g (12.0%) |
| Magnesium stearate | 0.3g (0.6%) | 0.3g (0.6%) | 0.3g (0.6%) | 0.2g (0.4%) | 0.3g (0.6%) | 0.2g(0.4%) |
| Total | 50g | 50g | 50g | 50g | 50g | 50g |

Case 8 to 11 and Control Groups 5 and 6 were prepared according to the ratios listed in Table 7, with the preparation method the same as case 1.

The results were shown in Table 8. Case 8 to 11 had optimal RSD values for tablet hardness, tablet weight variations, oral disintegration time, and sensory evaluation. Control Group 5 and 6 had larger tablet weight variations and longer disintegration times, making their excipient ratios unsuitable.

**Table 8 The experimental results of case 8-11**

| | **Case 8** | **Case 9** | **Case 10** | **Case 11** | **Control 5** | **Control 6** |
|---|---|---|---|---|---|---|
| Hardness | 34N | 46N | 30N | 35N | 45N | 43N |
| RSD value of hardness | 5.6% | 4.8% | 4.3% | 5.3% | 7.0% | 6.8% |
| Tablet weight variation | ±1.8% | 2.1% | ±1.8% | ±1.5% | ±8.8% | ±8.2% |
| Disintegration time | 42S | 70S | 75S | 55S | 160S | 200S |
| Oral disintegration time | 55S | 82S | 83S | 67S | 184S | 235S |
| Oral dryness | None | None | None | None | None | None |
| Oral gritty sensation | None | None | None | None | None | None |
| Sweet taste | Moderate | Moderate | Moderate | Moderate | Moderate | Moderate |

### Example 5: Study on Freeze-Drying Process of Recombinant Dust Mite Group 2 Allergen and Preparation of Sublingual Tablets

### 1) Freeze-Drying Process Study

Condition 1: The recombinant Der f 2 protein solution was exchanged into 50 mM PB + 3% mannitol, pH 7.0 buffer system, and the protein concentration was determined using BCA Protein Concentration Assay Kit to reach 1.0 mg/ml. The solution was poured into a freeze-drying dish, with the liquid level height ≤1cm. Freeze-drying was performed using a vacuum freeze dryer (LY0-21SP0IP, Dongfulong) according to the parameters in Tables 9-12 (Freeze-Drying Process 1-4) and Table 15 (Freeze-Drying Control 1) to prepare the freeze-dried powder.

Condition 2: The recombinant Der f 2 protein solution was exchanged into 5 mM PB + 1% mannitol + 1.5 mg/ml 150LB gelatin (bovine-derived, purchased from Baotou Dongbao Biotechnology Co., Ltd.), pH 7.0 buffer system, and the protein concentration was determined to reach 0.03 ± 0.015 mg/ml. The solution was poured into a freeze-drying dish, with the liquid level height ≤1cm. Freeze-drying was performed according to the parameters in Tables 13, 14, 16, and 17 (Freeze-Drying Process 5, 6, and Freeze-Drying Control 2, 3) using a vacuum freeze dryer to prepare the freeze-dried powder. The gelatin used in this example was bovine-derived, purchased from Baotou Dongbao Biotechnology Co., Ltd. It was verified that gelatin of different sources and specifications had no significant differences in the moisture content, protein recovery rate, uniformity, and appearance of the freeze-dried powder.

The set time refers to the time taken for the freeze-dryer to reach the set temperature, and the duration time refers to the time that the freeze-dryer maintains the set temperature.

**Table 9 Freeze-drying process 1**

| **Process** | **Temperature** | **Set Time** | **Duration** | **Vacuum** |
|---|---|---|---|---|
| Freezing | -12° C | 60min | 60min | / |
| | -50° C | 90min | 120min | / |
| First drying | -15° C | 360min | 960min | 0.18~0.2mbar |
| Secondary drying | 25° C | 300min | 480min | 0.18~0.2mbar |

**Table 10 Freeze-drying process 2**

| **Process** | **Temperature** | **Set Time** | **Duration** | **Vacuum** |
|---|---|---|---|---|
| Freezing | -15° C | 90min | 90min | / |
| | -45° C | 60min | 180min | / |
| First drying | -20° C | 360min | 960min | 0.18~0.2mbar |
| Secondary drying | 20° C | 480min | 480min | 0.18~0.2mbar |

**Table 11 Freeze-drying process 3**

| **Process** | **Temperature** | **Set Time** | **Duration** | **Vacuum** |
|---|---|---|---|---|
| Freezing | -12° C | 70min | 80min | / |
| | -50° C | 60min | 120min | / |
| First drying | -10° C | 380min | 840min | 0.18~0.2mbar |
| Secondary drying | 25° C | 300min | 480min | 0.18~0.2mbar |

**Table 12 Freeze-drying process 4**

| **Process** | **Temperature** | **Set Time** | **Duration** | **Vacuum** |
|---|---|---|---|---|
| Freezing | -12° C | 60min | 60min | / |
| | -50° C | 60min | 120min | / |
| First drying | -15° C | 360min | 840min | 0.18~0.2mbar |
| Secondary drying | 25° C | 300min | 600min | 0.18~0.2mbar |

**Table 13 Freeze-drying process 5**

| **Process** | **Temperature** | **Set Time** | **Duration** | **Vacuum** |
|---|---|---|---|---|
| Freezing | -12° C | 60min | 60min | / |
| | -50° C | 60min | 800min | / |
| First drying | -25° C | 360min | 900min | 0.18~0.2mbar |
| Secondary drying | 25° C | 300min | 600min | 0.18~0.2mbar |

**Table 14 Freeze-drying process 6**

| **Process** | **Temperature** | **Set Time** | **Duration** | **Vacuum** |
|---|---|---|---|---|
| Freezing | -12° C | 60min | 60min | / |
| | -50° C | 60min | 900min | / |
| First drying | -25° C | 360min | 900min | 0.18~0.2mbar |
| Secondary drying | 25° C | 300min | 1500min | 0.18~0.2mbar |

**Table 15 Freeze-drying process Control 1**

| **Process** | **Temperature** | **Set Time** | **Duration** | **Vacuum** |
|---|---|---|---|---|
| Freezing | -12° C | 60min | 60min | / |
| | -50° C | 60min | 120min | / |
| First drying | -25° C | 180min | 960min | 0.18~0.2mbar |
| Secondary drying | 25° C | 300min | 480min | 0.18~0.2mbar |

**Table 16 Freeze-drying process Control 2**

| **Process** | **Temperature** | **Set Time** | **Duration** | **Vacuum** |
|---|---|---|---|---|
| Freezing | -12° C | 60min | 60min | / |
| | -50° C | 60min | 120min | / |
| First drying | -25° C | 360min | 960min | 0.18~0.2mbar |
| Secondary drying | 25° C | 300min | 480min | 0.18~0.2mbar |

**Table 17 Freeze-drying process Control 3**

| **Process** | **Temperature** | **Set Time** | **Duration** | **Vacuum** |
|---|---|---|---|---|
| Freezing | -12° C | 60min | 60min | / |
| | -50° C | 60min | 360min | / |
| First drying | -25° C | 360min | 900min | 0.18~0.2mbar |
| Secondary drying | 25° C | 300min | 480min | 0.18~0.2mbar |

The results obtained from different freeze-drying processes were shown in Table 18. Freeze-Drying Process 1-4 had moisture contents ≤3.0% and uniformity ≤1%, and the process was suitable, and Process 4 was the optimal. Freeze-Drying Process 5-6 had moisture contents ≤3.5% and uniformity ≤1%, and the process was suitable too. Freeze-Drying Control 1 and 2 had relatively high moisture contents, and the process was not suitable. Freeze-Drying Control 3 had unacceptable appearance, and the process was not suitable too.

**Table 18 Test results of freeze-dried powder obtained by different freeze-drying processes**

| Freeze-drying process | Moisture content (%) | Protein content (mg/mg) | Uniformity (RSD value) | Appearance |
|---|---|---|---|---|
| Freeze-drying process 1 | 2.1% | 0.02586 | 1.0% | Qualified |
| Freeze-drying process 2 | 3.0% | 0.02480 | 0.8% | Qualified |
| Freeze-drying process 3 | 2.5% | 0.02507 | 0.8% | Qualified |
| Freeze-drying process 4 | 2.0% | 0.02507 | 0.4% | Qualified |
| Freeze-drying process 5 | 3.4% | 0.001973 | 0.8% | Qualified |
| Freeze-drying process 6 | 3.0% | 0.001989 | 0.5% | Qualified |
| Freeze-drying process control 1 | 16.6% | 0.02375 | 1.1% | Qualified |
| Freeze-drying process control 2 | 9.5% | 0.02454 | 1.5% | Qualified |
| Freeze-drying process control 3 | - | - | - | Unqualified |

### 2) Excipient Compatibility Study

The freeze-dried powder obtained from Freeze-Drying Process 4 was ground and sieved through a 30-mesh sieve. Combinations were prepared according to Table 19, and excipient compatibility studies were conducted through accelerated, light, and high-humidity tests.

**Table 19 Ratios for Der f2 compatibility studies**

| **Number** | **Ingredients** | | **Content** | |
|---|---|---|---|---|
| | A | B | A | B |
| ① | Freeze-Dried Powder | / | 10mg | / |
| ② | Freeze-Dried Powder | Lactose | 10mg | 50mg |
| ③ | Freeze-Dried Powder | Mannitol | 10mg | 50mg |
| ④ | Freeze-Dried Powder | Sorbitol | 10mg | 50mg |
| ⑤ | Freeze-Dried Powder | Microcrystalline cellulose | 10mg | 50mg |
| ⑥ | Freeze-Dried Powder | LMC | 10mg | 50mg |
| ⑦ | Freeze-Dried Powder | L-HPC | 10mg | 50mg |
| ⑧ | Freeze-Dried Powder | Croscarmellose sodium | 10mg | 50mg |
| ⑨ | Freeze-Dried Powder | Carboxymethyl starch sodium | 10mg | 50mg |
| ⑩ | Freeze-Dried Powder | Magnesium stearate | 10mg | 10mg |
| ⑪ | Freeze-Dried Powder | Glyceryl behenate | 10mg | 10mg |

The excipient compatibility test results were shown in Table 20, indicating that lactose, mannitol, low-moisture microcrystalline cellulose, low-substituted hydroxypropyl cellulose, magnesium stearate, and glyceryl behenate (with magnesium stearate slightly superior to glyceryl behenate) exhibited relatively good compatibility with the freeze-dried powder of recombinant Der f 2 protein. Sorbitol, microcrystalline cellulose, croscarmellose sodium, and carboxymethyl starch sodium had poor compatibility with the freeze-dried powder of recombinant Der f 2 protein. It was verified that the compatibility results of freeze-dried powders obtained from Freeze-Drying Processes 1-6 with the above excipients were similar.

**Table 20 Results of the Der f 2 compatibility study**

| **Conditions** | **Test** | ① | ② | ③ | ④ | ⑤ | ⑥ | ⑦ | ⑧ | ⑨ | ⑩ | ⑪ |
|---|---|---|---|---|---|---|---|---|---|---|---|---|
| Accelerated Conditions 5 days | Purity | 98% | 99% | 99% | 83% | 92% | 97% | 96% | 74% | 82% | 96% | 94% |
| Accelerated Conditions 10 days | Purity | 98% | 97% | 97% | 72% | 85% | 96% | 95% | 72% | 80% | 93% | 92% |
| Photostability Conditions 5 days | Purity | 99% | 99% | 99% | 92% | 96% | 98% | 99% | 94% | 95% | 96% | 93% |
| Photostability Conditions 10 days | Purity | 99% | 99% | 99% | 78% | 94% | 98% | 98% | 93% | 93% | 94% | 92% |
| High Humidity Conditions 5 days | Purity | 99% | 98% | 96% | 74% | 92% | 97% | 98% | 85% | 82% | 98% | 97% |
| High Humidity Conditions 10 days | Purity | 96% | 96% | 94% | 66% | 88% | 98% | 97% | 80% | 82% | 97% | 95% |

### 3) Preparation of Sublingual Tablets

Freeze-dried powder was prepared according to Condition 1 and Freeze-Drying Process 4. Based on the excipient compatibility study results, case 12 was prepared according to the formulation in Table 21.

**Table 21 Ratio of Case 12**

| **Ingredients** | **The formulation amount (Ratio)** |
|---|---|
| Freeze-Dried Powder | 0.37g (0.74%) |
| Lactose | 34.62g (69.25%) |
| Mannitol | 6.7g (13.40%) |
| L-HPC | 2g (4.00%) |
| LMC | 6g (12.00%) |
| Magnesium stearate | 0.3g (0.60%) |
| The total amount was 50g, and each tablet weighed 50mg | |

According to the ratio in Table 21, lactose was added layer by layer. Add and mix 5% lactose, freeze-dried powder, 5% lactose, mannitol, 5% lactose, low-substituted hydroxypropyl cellulose, 5% lactose, low-moisture microcrystalline cellulose, and the remaining lactose sequentially. Finally, magnesium stearate was added and mixed. A small single-punch tablet press was used to control the tablet hardness at 20-60N and tablet weight of 50mg.

It was confirmed that the freeze-drying process for recombinant Der f 2 protein was also applicable to recombinant Der p 2 protein. The results of excipient compatibility studies of Der p 2 were consistent with those of recombinant Der f 2 protein. The recombinant Der p 2 protein solution was freeze-dried (without gelatin), and the obtained freeze-dried powder was directly compressed into sublingual tablets using the powder direct compression method, designated as case 13. The preparation methods of the freeze-dried powder and sublingual tablets were the same as those of case 12.

### Example 6: Study on Freeze-Drying Process of Recombinant House Dust Mite Group 1 Allergen and Preparation of Sublingual Tablets

### 1. Study on Freeze-Drying Buffer and Protein Concentration

Condition 1: The recombinant Der f 1 protein solution was exchanged into 50mM acetic acid-sodium acetate pH 5.0+3% mannitol buffer system, and the protein concentration was adjusted to 0.1mg/ml. Freeze-drying was performed using a vacuum freeze-dryer (LY0-21SP0IP, Dongfulong) to prepare freeze-dried powder as Control Group 6.

Condition 2: The recombinant Der f 1 protein solution was exchanged into 50mM acetic acid-sodium acetate pH 5.0+3% mannitol buffer system, and the protein concentration was adjusted to 0.5mg/ml. Freeze-drying was performed using the same vacuum freeze-dryer to prepare freeze-dried powder as Control Group 7.

Condition 3: The recombinant Der f 1 protein solution was exchanged into 50mM citric acid-sodium citrate pH 5.0+3% mannitol buffer system, and the protein concentration was adjusted to 0.1mg/ml. Freeze-drying was performed to prepare freeze-dried powder as Control Group 8.

Condition 4: The recombinant Der f 1 protein solution was exchanged into 50mM citric acid-sodium citrate pH 5.0+3% mannitol buffer system, and the protein concentration was adjusted to 0.5mg/ml. Freeze-drying was performed to prepare freeze-dried powder as Control Group 9.

Condition 5: The recombinant Der f 1 protein solution was exchanged into 50mM citric acid-sodium citrate pH 5.0+3% mannitol buffer system, and the protein concentration was adjusted to 1.0mg/ml. Freeze-drying was performed to prepare freeze-dried powder as Control Group 10.

Condition 6: The recombinant Der f 1 protein solution was exchanged into 5mM citric acid-sodium citrate+1% mannitol+2mg/ml 150LB gelatin, pH 5.0 buffer system, and the protein concentration was adjusted to 0.04±0.02mg/ml. Freeze-drying was performed to prepare freeze-dried powder as Control Group 11. The gelatin used was bovine-derived and purchased from Baotou Dongbao Biotechnology Co., Ltd. It was verified that gelatin of different sources and specifications had no significant differences in the moisture content, protein recovery rate, uniformity, and appearance of the freeze-dried powder.

It was confirmed that the freeze-drying process for recombinant Der f 2 protein is also applicable to recombinant Der f 1 protein. The freeze-drying parameters for Conditions 1-5 in this example were the same as those in Freeze-Drying Process 4 of Example 5, and the freeze-drying parameters for Condition 6 were the same as those in Freeze-Drying Process 6 of Example 5.

After placing Control Groups 6-11 under accelerated conditions for 24h, the results were shown in Table 22. It can be confirmed that the lyophilization effect of Der f 1 at low concentration in the acetic acid-sodium acetate pH5.0+3% mannitol system was poor (Control Groups 6, 7), with high moisture content. The lyophilization effect of Der f1 at the concentration of 0. 5mg/ml and 1.0mg/ml in 50mM citric acid-sodium citrate pH 5.0 + 3% mannitol buffer (Control Groups 8, 9, 10) was relatively optimal, with 0.5mg/ml as the optimal condition.

The stability of Der f 1 freeze-dried powder which was freeze-dried in the 5mM citric acid-sodium citrate pH 5.0+1% mannitol buffer with 2mg/ml 150LB gelatin (Control Group 11) was good.

**Table 22 Study results of the Der f 1 freeze-drying system**

| **Conditions** | **Test** | **Control 6** | **Control 7** | **Control 8** | **Control 9** | **Control 10** | **Control 11** |
|---|---|---|---|---|---|---|---|
| 0h | Purity | 99% | 99% | 99% | 99% | 99% | 99% |
| | Potency | 100% | 100% | 100% | 100% | 100% | 100% |
| | Water content | 5.2% | 5.0% | 2.9% | 2.8% | 3.2% | 3.2% |
| | Protein content | 0.00183 | 0.00952 | 0.00197 | 0.01141 | 0.02187 | 0.002203 |
| | Uniformity | 3.3% | 3.8% | 6.0% | 1.0% | 1.3% | 1.3% |
| Accelerated Conditions for 24h | Purity | - | - | 76% | 99% | 96% | 99% |
| | Potency | - | - | - | 100% | 89% | 100% |

### 2. Study on Excipient Compatibility

The freeze-dried powder from Control Group 9 was ground and sieved through a 30-mesh sieve. Mix according to Table 23, and excipient compatibility studies were conducted through accelerated, light, and high humidity tests.

**Table 23 Ratios for Der f 1 compatibility studies**

| **Number** | **Ingredients** | | **Content** | |
|---|---|---|---|---|
| | A | B | A | B |
| ① | Freeze-Dried Powder | / | 10mg | / |
| ② | Freeze-Dried Powder | Lactose | 10mg | 50mg |
| ③ | Freeze-Dried Powder | Mannitol | 10mg | 50mg |
| ④ | Freeze-Dried Powder | LMC | 10mg | 50mg |
| ⑤ | Freeze-Dried Powder | L-HPC | 10mg | 50mg |
| ⑥ | Freeze-Dried Powder | Croscarmellose sodium | 10mg | 50mg |
| ⑦ | Freeze-Dried Powder | Magnesium stearate | 10mg | 10mg |
| ⑧ | Freeze-Dried Powder | Glyceryl behenate | 10mg | 10mg |

The results of the excipient compatibility tests showed that lactose, mannitol, low-moisture microcrystalline cellulose, low-substituted hydroxypropyl cellulose, and magnesium stearate had relatively good compatibility with the freeze-dried powder of recombinant Der f 1 protein. In addition, the compatibility of the above excipients with freeze-dried powder of other allergen protein was also excellent. Croscarmellose sodium and glyceryl behenate had poor compatibility with the protein freeze-dried powder.

**Table 24 Results of Der f 1 compatibility studies**

| **Conditions** | **Test** | **①** | **②** | **③** | **④** | **⑤** | **⑥** | **⑦** | **⑧** |
|---|---|---|---|---|---|---|---|---|---|
| Accelerated Conditions 5 days | Purity | 85% | 85% | 84% | 85% | 84% | 80% | 84% | 82% |
| Accelerated Conditions 10 days | Purity | 84% | 83% | 83% | 83% | 84% | 76% | 82% | 78% |
| Photostability Conditions 5 days | Purity | 85% | 85% | 84% | 85% | 85% | 83% | 85% | 83% |
| Photostability Conditions 10 days | Purity | 85% | 83% | 83% | 85% | 84% | 80% | 84% | 79% |
| High Humidity Conditions 5 days | Purity | 85% | 84% | 84% | 84% | 84% | 80% | 84% | 83% |
| High Humidity Conditions 10 days | Purity | 83% | 84% | 84% | 83% | 84% | 78% | 84% | 79% |

### 3. Preparation of Sublingual Tablets

Using the freeze-dried powder from Control Group 9, case 14 was prepared according to the formulation in Table 25. The preparation method of the sublingual tablets was the same as that of case 12.

**Table 25 Ratio of case 14**

| **Ingredients** | **The formulation amount (Ratio)** |
|---|---|
| Freeze-Dried Powder | 0.87g (1.74%) |
| Lactose | 34.13g (68.26%) |
| Mannitol | 6.7g (13.40%) |
| L-HPC | 2g (4.00%) |
| LMC | 6g (12.00%) |
| Magnesium stearate | 0.3g (0.60%) |
| The total amount was 50g, and each tablet weighed 50mg | |

It was confirmed that the freeze-drying process for recombinant Der f 1 protein is applicable to recombinant Der p 1 protein. The results of excipient compatibility studies were consistent with those of recombinant Der f 1 protein.

The recombinant Der p 1 protein solution was freeze-dried (without gelatin), and the obtained freeze-dried powder was directly compressed into sublingual tablets using the powder direct compression method, designated as case 15. The preparation methods of the freeze-dried powder and sublingual tablets were the same as those of case 14.

### Example 7: Preparation ofsublingual tablets containing Der p 1 and Der f 2 without gelatin

Recombinant Der f 2 and Der p 1 were separately freeze-dried. The freeze-drying method for recombinant Der f 2 was the same as Condition 1 and Freeze-Drying Process 2 in Example 5, and the freeze-drying method for recombinant Der p 1 was the same as Condition 4 and Freeze-Drying Process 4 in Example 6. The obtained freeze-dried powders were separately crushed, sieved through a 30-mesh sieve, and the protein content was detected. Case 16 was prepared according to Table 26, containing two proteins. Lactose was added layer by layer. Add and mix 5% lactose, freeze-dried powder of recombinant Der f 2, 5% lactose, freeze-dried powder of recombinant Der f 1, 5% lactose, mannitol, 5% lactose, low-substituted hydroxypropyl cellulose, 5% lactose, low-moisture microcrystalline cellulose, and the remaining lactose sequentially. Finally, magnesium stearate was added and mixed. A small single-punch tablet press was used to control the tablet hardness at 20-60N and tablet weight of 50mg.

**Table 26 Ratios for case 16 containing Der p 1 and Der f 2**

| **Ingredients** | **The formulation amount (Ratio)** |
|---|---|
| rDer f 2 freeze-dried powder | 4.85g (9.7%) |
| rDer p 1 freeze-dried powder | 1.8g (3.6%) |
| Lactose | 30.65g (61.3%) |
| Mannitol | 4.15g (8.30%) |
| L-HPC | 2.0g (4.00%) |
| LMC | 6.25g (12.50%) |
| Magnesium stearate | 0.3g (0.60%) |
| The total amount was 50g, and each tablet weighed 50mg | |

### Example 8: Preparation of Sublingual Tablets Containing Der p 1 and Der f 2 Proteins with Different Types of Gelatin

Recombinant Der f 2 and Der p 1 were separately freeze-dried. The freeze-drying method for Der f 2 was the same as Condition 2 and Freeze-Drying Process 6 in Example 5, and the freeze-drying method for Der p 1 was the same as Condition 6 and Freeze-Drying Process 5 in Example 6. The freeze-dried powders were separately crushed, sieved through a 30-mesh sieve, and then protein content was measured separately. Case 17 to 19 were prepared according to Table 27 with 180LB gelatin, 240LB gelatin, and 245LB gelatin, respectively. Specifically, 180LB and 240LB gelatins were bovine-derived gelatins purchased from Rousselot Gelatines, while 245LB gelatin wss pig-derived gelatin from Gelita AG. The preparation method followed Example 7.

**Table 27 Ratios for case 17-19 containing Der p 1 and Der f 2**

| **Ingredients** | **The formulation amount (Ratio)** |
|---|---|
| rDer f 2 freeze-dried powder containing gelatin | 0.33g (0.66%) |
| rDer p 1 freeze-dried powder containing gelatin | 0.54g (1.08%) |
| Lactose | 36.43g (72.86%) |
| Mannitol | 4.15g (8.30%) |
| L-HPC | 2.0g (4.00%) |
| LMC | 6.25g (12.50%) |
| Magnesium stearate | 0.3g (0.60%) |
| The total amount was 50g, and each tablet weighed 50mg | |

### Example 9: Preparation of Sublingual Tablets Containing Der p 1, Der p 2, Der f 1, and Der f 2 Proteins without Gelatin

The recombinant Der f 2, Der p 2, Der f 1, and Der p 1 were individually freeze-dried, with Der f 2 and Der p 2 following the same freeze-drying method as Condition 1 and Freeze-Drying Process 3 in Example 5, and Der f 1 and Der p 1 following Condition 4 and Freeze-Drying Process 4 in Example 6. The freeze-dried powders were crushed, sieved through a 30-mesh sieve, and the protein content was determined. Case 20 to 25 containing the four proteins were prepared according to Tables 28-33. Case 20 and 21 followed the same method as Example 7. In case 22-25, lactose was added layer by layer. Add and mix 5% lactose, low-substituted hydroxypropyl cellulose, 5% lactose, the freeze-dried powders of Der f 2, Der f 1, Der p 2, and Der p 1 sequentially, 5% lactose, low-moisture microcrystalline cellulose, mannitol, and the remaining lactose in a three-dimensional mixer. Magnesium stearate was added last. Tablets were pressed using a rotary tablet press with tablet hardness of 20-60N and weight of 50mg. The addition sequence and proportions of the four freeze-dried powders could be adjusted freely.

**Table 28 Ratio of case 20**

| **Ingredients** | **The formulation amount (Ratio)** |
|---|---|
| rDer f 2 freeze-dried powder | 0.16g (0.32%) |
| rDer p 2 freeze-dried powder | 0.16g (0.32%) |
| rDer f 1 freeze-dried powder | 0.072g (0.144%) |
| rDer p 1 freeze-dried powder | 0.072g (0.144%) |
| Lactose | 34.54g (69.07%) |
| Mannitol | 6.7g (13.40%) |
| L-HPC | 2g (4.00%) |
| LMC | 6g (12.00%) |
| Magnesium stearate | 0.3g (0.60%) |
| The total amount was 50g, and each tablet weighed 50mg | |

**Table 29 Ratio of Case 21**

| **Ingredients** | **The formulation amount (Ratio)** |
|---|---|
| rDer f 2 freeze-dried powder | 0.047g (0.094%) |
| rDer p 2 freeze-dried powder | 0.047g (0.094%) |
| rDer f 1 freeze-dried powder | 0.022g (0.044%) |
| rDer p 1 freeze-dried powder | 0.022g (0.044%) |
| Lactose | 34.86g (69.72%) |
| Mannitol | 6.7g (13.40%) |
| L-HPC | 2g (4.00%) |
| LMC | 6g (12.00%) |
| Magnesium stearate | 0.3g (0.60%) |
| The total amount was 50g, and each tablet weighed 50mg | |

**Table 30 Ratio of case 22**

| **Ingredients** | **The formulation amount (Ratio)** |
|---|---|
| rDer f 2 freeze-dried powder | 2.521g (0.504%) |
| rDer p 2 freeze-dried powder | 1.345g (0.269%) |
| rDer f 1 freeze-dried powder | 0.152g (0.030%) |
| rDer p 1 freeze-dried powder | 0.160g (0.032%) |
| Lactose | 368.8g (73.76%) |
| Mannitol | 41.5g (8.30%) |
| L-HPC | 20.0g (4.00%) |
| LMC | 62.5g (12.50%) |
| Magnesium stearate | 3.0g (0.60%) |
| The total amount was 500g, and each tablet weighed 50mg | |

**Table 31 Ratio of Case 23**

| **Ingredients** | **The formulation amount (Ratio)** |
|---|---|
| rDer f 2 freeze-dried powder | 2.179g (0.436%) |
| rDer p 2 freeze-dried powder | 1.159g (0.232%) |
| rDer f 1 freeze-dried powder | 2.613g (0.523%) |
| rDer p 1 freeze-dried powder | 2.749g (0.550%) |
| Lactose | 364.3g (72.86%) |
| Mannitol | 41.5g (8.30%) |
| L-HPC | 20.0g (4.00%) |
| LMC | 62.5g (12.50%) |
| Magnesium stearate | 3.0g (0.60%) |
| The total amount was 500g, and each tablet weighed 50mg | |

**Table 32 Ratio of case 24**

| **Ingredients** | **The formulation amount (Ratio)** |
|---|---|
| rDer f 2 freeze-dried powder | 1.094g (0.219%) |
| rDer p 2 freeze-dried powder | 0.582g (0.116%) |
| rDer f 1 freeze-dried powder | 1.313g (0.263%) |
| rDer p 1 freeze-dried powder | 1.381g (0.276%) |
| Lactose | 368.7g (73.73%) |
| Mannitol | 41.5g (8.30%) |
| L-HPC | 20.0g (4.00%) |
| LMC | 62.5g (12.50%) |
| Magnesium stearate | 3.0g (0.60%) |
| The total amount was 500g, and each tablet weighed 50mg | |

**Table 33 Ratio of Case 25**

| **Ingredients** | **The formulation amount (Ratio)** |
|---|---|
| rDer f 2 freeze-dried powder | 0.366g (0.073%) |
| rDer p 2 freeze-dried powder | 0.194g (0.039%) |
| rDer f 1 freeze-dried powder | 0.439g (0.088%) |
| rDer p 1 freeze-dried powder | 0.461g (0.092%) |
| Lactose | 371.58g (74.31%) |
| Mannitol | 41.5g (8.30%) |
| L-HPC | 20.0g (4.00%) |
| LMC | 62.5g (12.50%) |
| Magnesium stearate | 3.0g (0.60%) |
| The total amount was 500g, and each tablet weighed 50mg | |

### Example 10: Preparation of Sublingual Tablets Containing Der p 1, Der p 2, Der f 1, and Der f 2 Proteins with Different Types of Gelatin

The recombinant Der f 2, Der p 2, Der f 1, and Der p 1 were individually freeze-dried, with Der f 2 and Der p 2 following the same preparation method as Condition 2 and Freeze-Drying Process 6 in Example 5, and Der f 1 and Der p 1 following Condition 6 and Freeze-Drying Process 5 in Example 6. The freeze-dried powders were crushed, sieved through a 30-mesh sieve, and the content was measured. Case 26-28 useed 150LB, 180LB, and 240LB gelatins, respectively, and the sublingual tablets were prepared according to Table 34. Case 29-31 were prepared according to Tables 35-37. The preparation method was the same as Example 7.

**Table 34 Ratio of case 26-28 using different gelatins**

| **Ingredients** | **The formulation amount (Ratio)** |
|---|---|
| rDer f 2 freeze-dried powder containing gelatin | 3.24g (6.48%) |
| rDer p 2 freeze-dried powder containing gelatin | 1.61g (3.22%) |
| rDer f 1 freeze-dried powder containing gelatin | 0.9g (1.8%) |
| rDer p 1 freeze-dried powder containing gelatin | 0.9g (1.8%) |
| Lactose | 30.65g (61.3%) |
| Mannitol | 4.15g (8.30%) |
| L-HPC | 2.1g (4.20%) |
| LMC | 6.25g (12.50%) |
| Magnesium stearate | 0.2g (0.40%) |
| The total amount was 50g, and each tablet weighed 50mg | |

**Table 35 Ratios of case 29**

| **Ingredients** | **The formulation amount (Ratio)** |
|---|---|
| rDer f 2 freeze-dried powder containing 180LB gelatin | 3.24g (6.48%) |
| rDer p 2 freeze-dried powder containing 180LB gelatin | 1.61g (3.22%) |
| rDer f 1 freeze-dried powder containing 180LB gelatin | 0.9g (1.8%) |
| rDer p 1 freeze-dried powder containing 180LB gelatin | 0.9g (1.8%) |
| Lactose | 32.3g (64.6%) |
| Mannitol | 2.50g (5.0%) |
| L-HPC | 2.0g (4.00%) |
| LMC | 6.25g (12.50%) |
| Magnesium stearate | 0.3g (0.60%) |
| The total amount was 50g, and each tablet weighed 50mg | |

**Table 36 Ratio of Case 30**

| **Ingredients** | **The formulation amount (Ratio)** |
|---|---|
| rDer f 2 freeze-dried powder containing 180LB gelatin | 3.24g (6.48%) |
| rDer p 2 freeze-dried powder containing 180LB gelatin | 1.61g (3.22%) |
| rDer f 1 freeze-dried powder containing 180LB gelatin | 0.9g (1.8%) |
| rDer p 1 freeze-dried powder containing 180LB gelatin | 0.9g (1.8%) |
| Lactose | 33.55g (67.1%) |
| Mannitol | 2.50g (5.0%) |
| L-HPC | 2.0g (4.00%) |
| LMC | 5.0g (10.0%) |
| Magnesium stearate | 0.3g (0.60%) |
| The total amount was 50g, and each tablet weighed 50mg | |

**Table 37 Ratio of case 31**

| **Ingredients** | **The formulation amount (Ratio)** |
|---|---|
| rDer f 2 freeze-dried powder containing 180LB gelatin | 3.24g (6.48%) |
| rDer p 2 freeze-dried powder containing 180LB gelatin | 1.61g (3.22%) |
| rDer f 1 freeze-dried powder containing 180LB gelatin | 0.9g (1.8%) |
| rDer p 1 freeze-dried powder containing 180LB gelatin | 0.9g (1.8%) |
| Lactose | 29.65g (59.3%) |
| Mannitol | 4.15g (8.3%) |
| L-HPC | 3.0g (6.00%) |
| LMC | 6.25g (12.5%) |
| Magnesium stearate | 0.3g (0.60%) |
| The total amount was 50g, and each tablet weighed 50mg | |

### Example 11: Preparation of Sublingual Tablets Containing Art a 1, Art a 3, Art v 1, and Art v 3 Proteins with Different Types of Gelatin

Art a 1, Art a 3, Art v 1, and Art v 3 were individually diluted to 0.06±0.03mg/ml in a buffer system of 10mM citric acid-sodium citrate + 1% mannitol + 3mg/ml 180LB gelatin at pH 5.0. Freeze-drying was performed using a vacuum freeze-dryer. Art a 1 and Art v 1 followed Freeze-Drying Process 6 in Example 5, while Art a 3 and Art v 3 followed Process 5. The freeze-dried powders were crushed, sieved through a 30-mesh sieve, and the content was determined. Case 32 and 33 were prepared according to Tables 38 and 39, following the same method as Example 7.

**Table 38 Ratio of case 32**

| **Ingredients** | **The formulation amount (Ratio)** |
|---|---|
| Art a 1 freeze-dried powder containing 180LB gelatin | 1.95g (3.9%) |
| Art a 3 freeze-dried powder containing 180LB gelatin | 1.95g (3.9%) |
| Art v 1 freeze-dried powder containing 180LB gelatin | 1.95g (3.9%) |
| Art v 3 freeze-dried powder containing 180LB gelatin | 1.95g (3.9%) |
| Lactose | 32.4g (64.8%) |
| Mannitol | 2.50g (5.0%) |
| L-HPC | 2.0g (4.00%) |
| LMC | 5.0g (10.0%) |
| Magnesium stearate | 0.3g (0.60%) |
| The total amount was 50g, and each tablet weighed 50mg | |

**Table 39 Ratio of case 33**

| **Ingredients** | **The formulation amount (Ratio)** |
|---|---|
| Art a 1 freeze-dried powder containing 180LB gelatin | 0.49g (0.98%) |
| Art a 3 freeze-dried powder containing 180LB gelatin | 0.49g (0.98%) |
| Art v 1 freeze-dried powder containing 180LB gelatin | 0.49g (0.98%) |
| Art v 3 freeze-dried powder containing 180LB gelatin | 0.49g (0.98%) |
| Lactose | 38.24g (76.48%) |
| Mannitol | 2.50g (5.0%) |
| L-HPC | 2.0g (4.00%) |
| LMC | 5.0g (10.0%) |
| Magnesium stearate | 0.3g (0.60%) |
| The total amount was 50g, and each tablet weighed 50mg | |

### Example 12: Testing of Sublingual Tablets of case 12-31

The sublingual tablets of case 12-33, containing one, two, or four proteins, with or without gelatin from different sources, exhibited low RSD values for tablet hardness, low weight variation, short disintegration times, and pleasant oral sensations without dryness, grittiness, and moderate sweetness. The uniformity of content was excellent. These tablets contained low percentages of different protein raw materials (0.276% in case 21 to 13.3% in case 16, 26-31) and rapidly disintegrated sublingually within 90 seconds. Even with low protein content (e.g., 0.74% in case 12 and 0.276% in case 21), the uniformity met the pharmacopoeial requirement of less than 15%. Partial results were shown in Table 40.

Since the protein content in the sublingual tablets was low, it was reasonable to expect that when other allergen proteins were used as active ingredients, the sublingual tablets will still have excellent RSD values for hardness, low weight variation, short disintegration times, pleasant oral sensations, and uniform content.

**Table 40: Testing Results for Tablets in case 12-19**

| | Case 12 | Case 13 | Case 14 | Case 15 | Case 16 | Case 17 | Case 18 | Case 19 |
|---|---|---|---|---|---|---|---|---|
| Hardness | 32N | 31N | 30N | 28N | 22N | 25N | 23N | 22N |
| RSD value of hardness | 4.3% | 3.9% | 5.2% | 5.4% | 2.8% | 3.0% | 2.8% | 3.2% |
| Tablet weight variation | ±1.0% | ±0.8% | ±1.6% | ±1.3% | ±1.5% | ±1.1% | ±2.3% | ±2.8% |
| Disintegration time | 35s | 29s | 28s | 30s | 20s | 18s | 68s | 68s |
| Uniformity of content (A+2.2S) | 9.06 | 8.83 | 8.84 | 9.18 | 11.28 | 12.53 | 12.84 | 11.67 |
| Oral disintegration time | 42s | 30s | 32s | 40s | 30s | 26s | 82s | 88s |
| Oral dryness | None | None | None | None | None | None | None | None |
| Oral sandy ensation | None | None | None | None | None | None | None | None |
| Sweet taste | Moderat e | Moderat e | Moderat e | Moderat e | Moderat e | Moderat e | Moderat e | Moderat e |

Long-term stability and accelerated stability tests were conducted on the sublingual tablets from case 16-19 containing two allergen proteins. The stability of tablets containing gelatin (case 17-19) under high temperature conditions was superior to that of tablets without gelatin (case 16), as evidenced by the stability of major allergen types I and II.

**Table 41 25°C Long-term Stability and Accelerated Stability Testing Results for Tablets in case 16-19**

| **Conditions** | **Test** | **Case 16** | **Case 17** | **Case 18** | **Case 19** |
|---|---|---|---|---|---|
| 0 | Content of type I major allergen | 100% | 102% | 105% | 102% |
| | Content of type II major allergen | 103% | 105% | 103% | 105% |
| 25° C 30 days | Content of type I major allergen | 102% | 100% | 105% | 100% |
| | Content of type II major allergen | 98% | 102% | 100% | 105% |
| Accelerated Conditions 30 days | Content of type I major allergen | 59% | 94% | 108% | 109% |
| | Content of type II major allergen | 46% | 80% | 78% | 77% |

Further, stability tests under 25°C, accelerated, light, and high humidity conditions were performed on sublingual tablets from case 23, 24, and 26-33 containing four allergen proteins. The stability of tablets containing gelatin (case 26-32) was superior to those without gelatin (case 23, 24) under high temperature, light, and high humidity conditions, as evidenced by the content of major allergen. Case 32 and 33 demonstrated excellent stability under 25°C and 40°C long-term storage. Results were presented in Tables 40 and 41.

**Table 40: Stability Testing Results for Dust Mite Tablets under 25°C, Accelerated, Light, and High Humidity Conditions**

| **Condition s** | **Test** | Case 23 | Case 24 | Case 26 | Case 27 | Case 28 | Case 29 | Case 30 | Case 31 |
|---|---|---|---|---|---|---|---|---|---|
| 0 day | Content of type I major allergen | 98% | 97% | 100% | 100% | 100% | 98% | 97% | 93% |
| | Content of type II major allergen | 103% | 116% | 100% | 100% | 100% | 103% | 116% | 115% |
| 25° C 60 days | Content of type I major allergen | 72% | 77% | 103% | 102% | 99% | 105% | 107% | 103% |
| | Content of type II major allergen | 92% | 90% | 94% | 105% | 93% | 101% | 105% | 96% |
| 25° C 90 days | Content of type I major allergen | 61% | 67% | 102% | 100% | 100% | 109% | 112% | 108% |
| | Content of type II major allergen | 82% | 80% | 100% | 106% | 103% | 101% | 118% | 103% |
| Accelerate d Conditions 5 days | Content of type I major allergen | 66% | 61% | 105% | 103% | 100% | 102% | 98% | 92% |
| | Content of type II major allergen | 59% | 65% | 93% | 100% | 95% | 103% | 104% | 102% |
| Accelerate d Conditions 10 days | Content of type I major allergen | 48% | 45% | 108% | 102% | 100% | 107% | 107% | 105% |
| | Content of type II major allergen | 47% | 38% | 82% | 98% | 80% | 105% | 101% | 101% |
| Photostabil ity Conditions 5 days | Content of type I major allergen | 75% | 76% | - | - | - | 91% | 95% | 97% |
| | Content of type II major allergen | 103% | 102% | - | - | - | 109% | 109% | 106% |
| Photostabil ity Conditions 10 days | Content of type I major allergen | 77% | 72% | - | - | - | 105% | 110% | 104% |
| | Content of type II major allergen | 99% | 96% | - | - | - | 104% | 105% | 102% |
| High Humidity Conditions 5 days | Content of type I major allergen | 64% | 61% | - | - | - | 102% | 107% | 106% |
| | Content of type II major allergen | 105% | 109% | - | - | - | 103% | 110% | 111% |
| High Humidity Conditions 10 days | Content of type I major allergen | 62% | 63% | - | - | - | 107% | 108% | 107% |
| | Content of type II major allergen | 103% | 109% | - | - | - | 105% | 96% | 104% |

**Table 41 Stability Assessment of Artemisia Pollen Tablets**

| **Conditions** | **Test** | **Case 32** | **Case 33** |
|---|---|---|---|
| 0 day | Content of type I major allergen | 100% | 100% |
| | Content of type III major allergen | 100% | 100% |
| 40° C 30 days | Content of type I major allergen | 121% | 108% |
| | Content of type III major allergen | 117% | 113% |
| 40° C 90 days | Content of type I major allergen | 100% | 106% |
| | Content of type III major allergen | 94% | 98% |
| 25° C 90 days | Content of type I major allergen | 101% | 109% |
| | Content of type III major allergen | 106% | 115% |
| | Content of type III major allergen | 103% | 109% |

Additionally, stability tests on sublingual tablets containing a single allergen protein (Der p 1, Der p 2, Der f 1, Der f 2) with or without gelatin revealed that tablets with gelatin had superior stability compared to those without gelatin.

## Claims

1. A sublingual tablet, comprising a protein raw material, lactose, mannitol, low substituted hydroxypropyl cellulose, low moisture microcrystalline cellulose, and magnesium stearate, and mass percentages of the protein raw material, the lactose, the mannitol, the low substituted hydroxypropyl cellulose, the low moisture microcrystalline cellulose, and the magnesium stearate are 0%-15.6%, 0%-83.4%, 0-83.4%, 4%-6%, 10%-15%, and 0.4%-0.6%, respectively.

2. The sublingual tablet according to claim 1, wherein the protein raw material is an allergen protein raw material.

3. The sublingual tablet according to claim 1, wherein the mass percentages of the protein raw material, the lactose, the mannitol, the low substituted hydroxypropyl cellulose, the low moisture microcrystalline cellulose, and the magnesium stearate are 0%-15.6%, 50%-83.4%, 0%-33.4%, 4%-6%, 10%-15%, and 0.4%-0.6%, respectively.

4. The sublingual tablet according to claim 1, wherein the mass percentages of the protein raw material, the lactose, the mannitol, the low substituted hydroxypropyl cellulose, the low moisture microcrystalline cellulose, and the magnesium stearate are 0.276%-15.6%, 59.3%-76.48%, 5%-13.4%, 4%-6%, 10%-12.5%, and 0.4%-0.6%, respectively.

5. The sublingual tablet according to claim 1, wherein the protein raw material is a dust mite allergen protein, and the dust mite allergen protein comprises one or a mixture of two or more selected from the group consisting of a Der p 1 raw material, a Der p 2 raw material, a Der f 1 raw material, and a Der f 2 raw material.

6. The sublingual tablet according to claim 1, wherein the protein raw material is an artemisia pollen allergen protein, and the artemisia pollen allergen protein comprises one or a mixture of two or more selected from the group consisting of an Art a 1 raw material, an Art a 3 raw material, an Art v 1 raw material, and an Art v 3 raw material.

7. The sublingual tablet according to any one of claims 1 to 6, wherein the protein raw material is a freeze-dried powder of an allergen protein.

8. The sublingual tablet according to claim 7, wherein the freeze-dried powder is supplemented with or without gelatin.

9. The sublingual tablet according to claim 7, wherein a buffer for freeze-drying the allergen protein contains citric acid-sodium citrate and mannitol, or contains PB buffer and mannitol.

10. The sublingual tablet according to any one of claims 1 to 9, wherein the freeze-dried powder is prepared by the following steps:
replacing a solution containing the allergen protein to a suitable buffer at a suitable pH condition, quantifying to an appropriate concentration, followed by pre-freezing, first drying, and secondary drying to obtain the freeze-dried powder.
